# EUROPEAN PATENT SPECIFICATION

(11) **EP 0 888 107 B1**
(45) Date of publication and mention of the grant of the patent: **09.04.2003**
(21) Application number: 97907658.5
(22) Date of filing: 19.02.1997
(51) Int. Cl.: A61K 6/02

(54) **LOW-FUSING TEMPERATURE PORCELAIN, COMPOSITIONS, PROSTHESES, METHODS AND KITS**
NIEDRIGSCHMELZENDES PORZELLAN, ZUSAMMENSETZUNGEN, PROTHESEN, VERFAHREN UND KITS
PORCELAINES A BASSE TEMPERATURE DE FUSION, COMPOSITIONS, PROTHESES, PROCEDES ET NECESSAIRES CORRESPONDANTS

(30) Priority: 23.02.1996 US 12125 P; 10.06.1996 US 660996
(43) Date of publication of application: 07.01.1999
(73) Proprietor: CERAMCO, INC., Burlington, NJ 08016 (US)
(72) Inventor: KRAMER PRIMUS, Carolyn, M., Sarasota, FL 34238 (US); McLAUGHLIN, John, F., Lakehurst, NJ 08733 (US); DeLUCA, Robert, D., Pennington, NJ 08543 (US); HORNOR, John, A., Siklerville, NJ 08081 (US); DAUB, Mary, J., Mt. Laurel, NJ 08054 (US); ANDINO, Kevin, A., Muskegon, MI 49443 (US)
(74) Representative: Hartz, Nikolai, Dr.
(86) International application number: US9702467
(87) International publication number: WO97030678

(56) References cited:
- EP-A- 0 478 937
- EP-A- 0 544 145
- EP-A- 0 630 639
- EP-A- 0 686 378
- EP-A- 0 695 726
- WO-A-96/18373
- DE-A- 3 911 460
- DE-A- 4 207 180
- NL-A- 9 200 564
- US-A- 4 170 823

## Description

The invention relates to porcelain compositions, dental prostheses, methods of use and kits thereof. More particularly the invention provides low-fusing temperature porcelain compositions, methods of use thereof on metal or porcelain substrates to form dental prostheses and kits therefor. The invention provides low-fusing porcelain paste opaque, low-fusing porcelain powder opaque, low-fusing porcelain crystals, low-fusing porcelain dentin and low-fusing porcelain transparent and opal enamels and low-fusing modifier porcelains including stains. The invention provides low-fusing porcelain having fluorescence, opalescence and which hold their shape when heated to temperatures between 750°C and 800°C. Low-fusing porcelain in accordance with a preferred embodiment of the invention is polishable to high luster, and the smoothness of the polished porcelain results in at least about 50 percent to 90 percent less wear on natural tooth enamel than polished coventional porcelain. Preferably an angle from a line tangent to a point on a surface contour of a coating of porcelain composition of the invention has less than a 20 percent change during heating from 23°C to 800°C. Preferably a physical surface feature of a dental prosthesis formed from a low-fusing temperature porcelain composition of the invention having a diameter (or longest dimension) of 0.5 mm retains a diameter (or longest dimension) of at least 0.25 mm after firing the prosthesis by heating from 23°C to 800°C and cooling from 800°C to 23°C five times.

A dental prosthesis often comprises a crown or bridge restoration in which a dental porcelain is fused to a supporting metal or ceramic substrate. Dental porcelains are generally a mixture of glass, ceramic opacifiers, pigments and the like. There are a number of key considerations in selecting glass frit components to form a porcelain that will be compatible with the selected substrate. For example, the porcelain must have a coefficient of thermal expansion close to but less - than that of the substrate. Otherwise, a porcelain fused to the substrate will tend to crack and separate from its supporting structure.

EP-A-0 544 145 and WO 96/18373 disclose a low-fusing temperature porcelain composition comprising SiO₂, Al₂O₃, Na₂O, K₂O, Li₂O, CaO, BaO, and CeO₂.

It is an object of the invention to provide a porcelain composition that has a low-fusing or processing temperature and a coefficient of thermal expansion such that it can be used in combination with both metal and porcelain substrates in dental prostheses, useful in making, repairing or improving crowns and bridges for both coventional restorations to natural dentition and to implant-based. restorations.

A low-fusing temperature porcelain system for use in a dental prosthesis is provided. The porcelain system of the invention has components with compositions which include from 40 to 65 percent by weight SiO₂, from 6 to 12 percent by weight Al₂O₃, from 6 to 12 percent by weight Na₂O, from 5.5 to 10.5 percent by weight K₂O, from 1 to 3 percent by weight Li₂O, from 0.8 to 2.5 percent by weight CaO, from 0.5 to 4 percent by weight B₂O₃ and from 0.1 to 0.8 percent by weight CeO₂ according to claim 1. Preferably compositions of the invention include from 0.01 to 3.0 percent by weight P₂O₅.

A preferred embodiment of the invention provides a composition which includes from 40 to 47 percent by weight SiO₂, from 5 to 8 percent by weight Al₂O₃, from 6 to 12 percent by weight Na₂O, from 9.0 to 10.5 percent by weight K₂O, from 1 to 3 percent by weight Li₂O, from 0.8 to 3.0 percent by weight CaO, from 0.8 to 2.0 percent by weight B₂O₃ and from 0.3 to 1.0 percent by weight CeO₂.

The porcelain of the invention has a fusing temperature of less than 800°C and a coefficient of thermal expansion compatible with a substrate of metal or ceramic. Preferably, the coefficient of thermal expansion is from about 11.5 to 13.4 ppm °K⁻¹ [from 30 to 430°C], and more preferably 12.4 to 13.4 ppm °K⁻¹ [from 30 to 500°C]

The invention provides low-fusing temperature porcelain compositions, methods of use thereof on metal or porcelain substrates to form dental prostheses and kits therefor. The invention provides a low-fusing temperature porcelain system that is a mixture of oxides such that the resulting porcelain components have a fusing temperature of about 800°C or less and a coefficient of thermal expansion of 11.5 to 12.5 ppm °K⁻¹ [30 to 430°C]. The low-fusing porcelain may be employed in dental crowns, bridges, restorations and fixed dentures wherein it is fused to a substrate or coping of dental alloy copings, such as UltraCrown PD alloy distributed by Dentsply International (Ceramco), or fused to an appropriate all-ceramic substrate.

The compositional range for the low-fusing temperature dental porcelains of the invention, having processing temperatures of less than about 800°C and a coefficient of thermal expansion (CTE) of 11.6 to 12.6 ppm °K⁻¹ [30 to 430°C] is a mixture of oxides having from 40 to 65 percent by weight SiO₂, from 6 to 12 percent by weight Al₂O₃, from 6 to 12 percent by weight Na₂O, from 5.5 to 10.5 percent by weight K₂O, from 1 to 3 percent by weight Li₂O, from 0.8 to 2.5 percent by weight CaO, from 0.5 to 4 percent by weight B₂O₃ and from 0.1 to 0.8 percent by weight CeO₂.

Liquids containing 98 to 100 percent by weight water are preferably mixed with porcelain compositions of the invention in from a one to one weight ratio to a one (part liquid) to four (parts porcelain) weight ratio and more preferably in a weight ratio of from one (part liquid) to three (parts porcelain). For example, liquids contain 0.1 percent by weight of salts and/or preferably less than 0.5 percent by weight of organic liquids and/or less than 0.5 percent by weight solids dissolved in water are preferably mixed with porcelain compositions of the invention in a one (part liquid) to three (parts porcelain) weight ratio. Carving liquids for use with porcelain are preferred for use with compositions of the invention for their ability to improve the handling and carving of porcelain and to reduce slumping during build-up and complete burn off at low temperatures. Also, Finesse Opaque Modifier Liquid and Finesse Stain Liquid are preferred for use in mixtures with porcelain compositions of the invention.

The low-fusing porcelain compositions of the invention are preferred for use in conjunction with non-precious or noble or high noble alloys when used for porcelain-fused-to-metal crowns, bridges or dental prostheses. The low-fusing porcelain components of the system preferably contain mixtures of frits, opacifiers and pigments in varied quantities to create an assortment of materials with a variety of fusion temperatures, translucencies and colors.

The low-fusing porcelain system of the invention is useful for fabricating porcelain-fused-to-metal restorations. All of the components fuse at temperatures approximately 200°C lower than corresponding components of conventional dental porcelains such as Ceramco II and Ceramco II Silver porcelains. Preferably, each porcelain in the system has fluorescence. The use of these porcelains allows the dental technician to create a more aesthetic restoration at a lower temperature.

The low-fusing dentin and enamel porcelain compositions of the invention are also easier to polish and yield smoother and less abrasive surfaces than conventional porcelains. They contain fewer leucite crystals than conventional porcelains. The porcelain composition of the invention possess superior handling characteristics, making the creation of a restoration easier for the dental technician.

The compositions of the invention provide a lower fusing temperature than standard porcelain system. The compositions of the invention provide low-fusing opaque porcelains having a fusing temperature of 775°C-790°C, compared to 975°C for a standard dental porcelain. The compositions of the invention provide low-fusing dentin porcelains having a fusing temperature of from 700°C to 760°C more preferably from 720°C to 740°C, compared to 920°C-940°C for a standard dentin porcelain.

The compositions of the invention provide thermal expansion compatible with a wider range of alloy compositions than Ceramco® II porcelain. The compositions of the invention provide low-fusing porcelains having a thermal expansion coefficient of 12.1 ppm/°C at 430°C, compared to 12.5 ppm/°C at 500°C for Ceramco® II porcelain.

Compositions of the invention cause less wear against the opposing natural teeth (dentition) than standard porcelain systems. Low-fusing dentin porcelain of a preferred embodiment of the invention provides wear of 5.6 X 10⁻² mm² compared to 18.2 X 10⁻² mm² for Ceramco porcelain in a three body wear test. Low-fusing dentin porcelain of a preferred embodiment of the invention has a Vickers hardness of 520 kg/mm², compared to 620 kg/mm² for Ceramco II porcelain. The compositions of the invention when utilized in forming dental prostheses are characterized by having an easier polishability due to the reduced hardness compared to conventional porcelain compositions.

Examples 1-5 describe preparation and use of preferred compositions of the inventions shown in Table 1 and characteristics of the resulting dental porcelain qualities are shown in Table 2.

### Example 1

A low-fusing temperature porcelain having the composition indicated in Table 1, under Example 1, is made by combining and fusing oxides, then pulverizing and blending with opacifiers and pigments. The resulting porcelain has a fusing temperature of less than 800 °C and a CTE of about 12.1 ppm °K⁻¹ from 30°C to 430°C. Samples of the resulting material have the characteristics and properties shown in Table 2 for low-fusing powder opaque when fired according to the schedule of Table 3. The schedule for firing includes 3 minutes of drying, 3 minutes of preheating at 450° C and heating from 450°C to 785°C under a vacuum of 29 inches of Hg at a rate of 90°C per minute, at 765°C the vacuum is released and heating is continued to 785°C.

### Example 1A

Low-fusing porcelain compositions of Example 1A of the invention are made in the same manner as Example 1, except having the compositions indicated in Table 1, under Example 1A.

### Example 2

Low-fusing porcelain compositions of Example 2 of the invention are made in the same manner as Example 1, except having the compositions indicated in Table 1, under Example 2.

### Example 2A

The procedure of Example 1 is followed except that the composition indicated in Table 1A under Example 2A is used in place of the composition in Table 1 under Example 1.

### Example 3

A dental prosthesis having porcelain coated on metal is made by mixing 3 parts of glass frit made as disclosed in Example 2 with one part of organic gel to form a paste. The gel is composed of glycerine, butylene glycol, diethylene glycol, preservative, cellulose, silica and water. The paste is then brushed onto metal base material to form a paste coating. 1 part of crystals made as disclosed in Table 1 under Example 3 are sprinkled onto the paste coating, for each 4 parts of paste coating, to form a paste with crystals embedded therein coating on the metal base. The crystal-embedded paste coating is then dried for 5 minutes, then preheated for 5 minutes at 450°C, then fired by heating to 785°C at a heating rate of 90°C per minute while under a vacuum of 29 inches of Hg between 450°C and 765°C and holding the temperature at 785 for 1 minute to form a light refractive surface opaque porcelain layer.

### Example 4

3 parts of a glass frit having the composition in Table 1 under Example 4 are mixed with 1 part of water to form a thick paste. The paste is brushed onto the outer surface of the opaque porcelain layer of Example 3.

### Example 4A and 4B

The procedure of Example 4 is followed except that the glass frit composition in Table 1A under Example 4A or 4B is used in place of that under Example 4 in Table 1.

### Example 5

The procedure of Example 4 is followed except that the glass frit used is disclosed in Table 1 under Example 5.

### DENTIN/ENAMEL LAYER

### Example 5A

To the product of Example 3, an opaque porcelain layer, on a conventional dental ceramic palladium-gold-silver alloy, is applied 2 parts of the product of Example 4 and then a discrete, separate addition of 1 part of the product of Example 5 and then drying at 100°C for 5 minutes, heating for 5 minutes at 450°C, followed by heating to 740°C at a heating rate of 35°C per minute while under a vacuum of 29 inches of Hg between 450°C and 720°C to form a dentin enamel layer on a crown.

### Example 5B

The procedure of example 4 is followed except that the glass frit used is that disclosed in Table 1 under Example 5B.

### Example 5C

The procedure of example 5A is followed, except that the glass frits of Examples 3 and 4 are replaced with the glass frits disclosed in Table 1 under Example 5B and 5C, respectively.

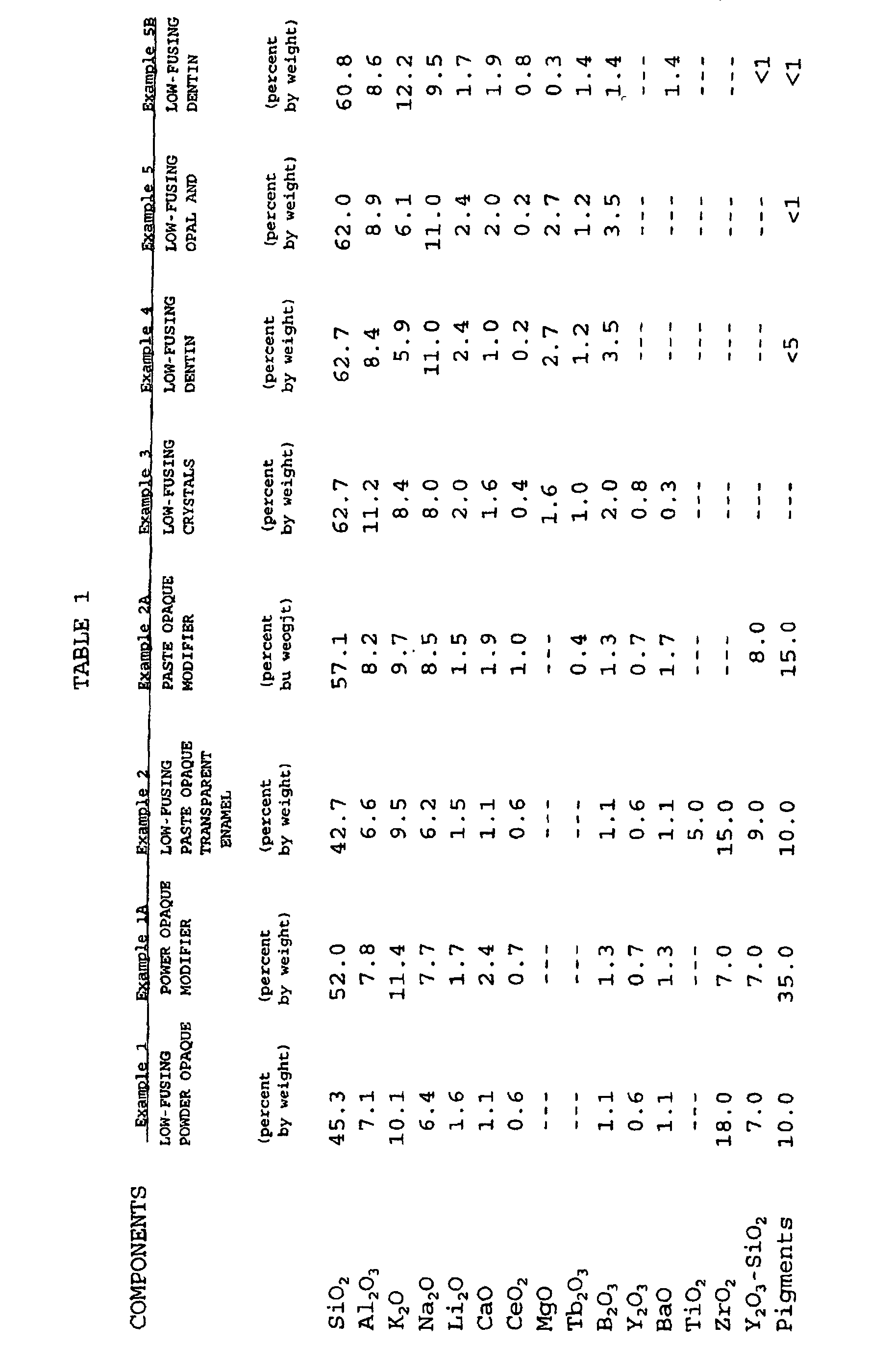

**TABLE 1A**

| COMPONENTS | Example 2A | Example 4A | Example 4B | Example 5C |
|---|---|---|---|---|
| | LOW-FUSING POWDER OPAQUE (percent by weight) | LOW-FUSING DENTIN (percent by weight) | LOW-FUSING DENTIN (percent by weight) | LOW-FUSING OPAL ENAMEL (percent by weight) |
| SiO₂ | 45.2 | 59 | 59 | 58.4 |
| Al₂O₃ | 6.1 | 7 | 7 | 8.0 |
| K₂O | 10.2 | 13.5 | 14.5 | 12.4 |
| Na₂O | 7.3 | 10.5 | 10.5 | 9.3 |
| Li₂O | 1.6 | 2 | 2 | 1.6 |
| CaO | 1.4 | 2 | 2 | 2.0 |
| CeO₂ | 0.7 | 1 | 1 | 1.1 |
| Tb₂O₃ | 0.7 | 1 | --- | 1.1 |
| B₂O₃ | 1.4 | 2 | --- | 1.5 |
| Y₂O₃ | --- | --- | 2 | --- |
| BaO | 1.4 | 2 | 2 | 1.8 |
| Pigments | (0-15) | <5 | <5 | <1 |
| ZrO₂ | 24.0 | <1 | <1 | <1 |
| P₂O₅ | | | | 2.8 |

**TABLE 2**

| | LOW-FUSING POWDER OPAQUE | LOW-FUSING PASTE OPAQUE | LOW-FUSING CRYSTALS | LOW-FUSING DENTIN | LOW-FUSING OPAL AND TRANSPARENT ENAMEL |
|---|---|---|---|---|---|
| Examples | 1 | 2 | 3 | 4 | 5 |
| Physical Property | | | | | |
| Flexural Strength (MPa) | 65 | 109 | - | 84 | 73 |
| Chemical Solubility* (µg/cm²) | 183 | 92 | - | 42 | 77 |
| | | | | | |
| Glass Transition Temperature (GTT) (°C) 2 firings | 460 | 500 | 486 | 465 | 464 |
| 4 firings | 464 | 500 | 484 | 462 | 461 |
| Thermal Expansion 25°C - GTT 2 firings (ppm/K) | 11.9 | 12.2 | 12.3 | 12.1 | 12.4 |
| Thermal Stability 4 firings (ppm/K) | 12.4 | 12.2 | 11.9 | 12.0 | 12.3 |

| | | | | | |
|---|---|---|---|---|---|
| *after 16 hours in boiling acetic acid. | | | | | |

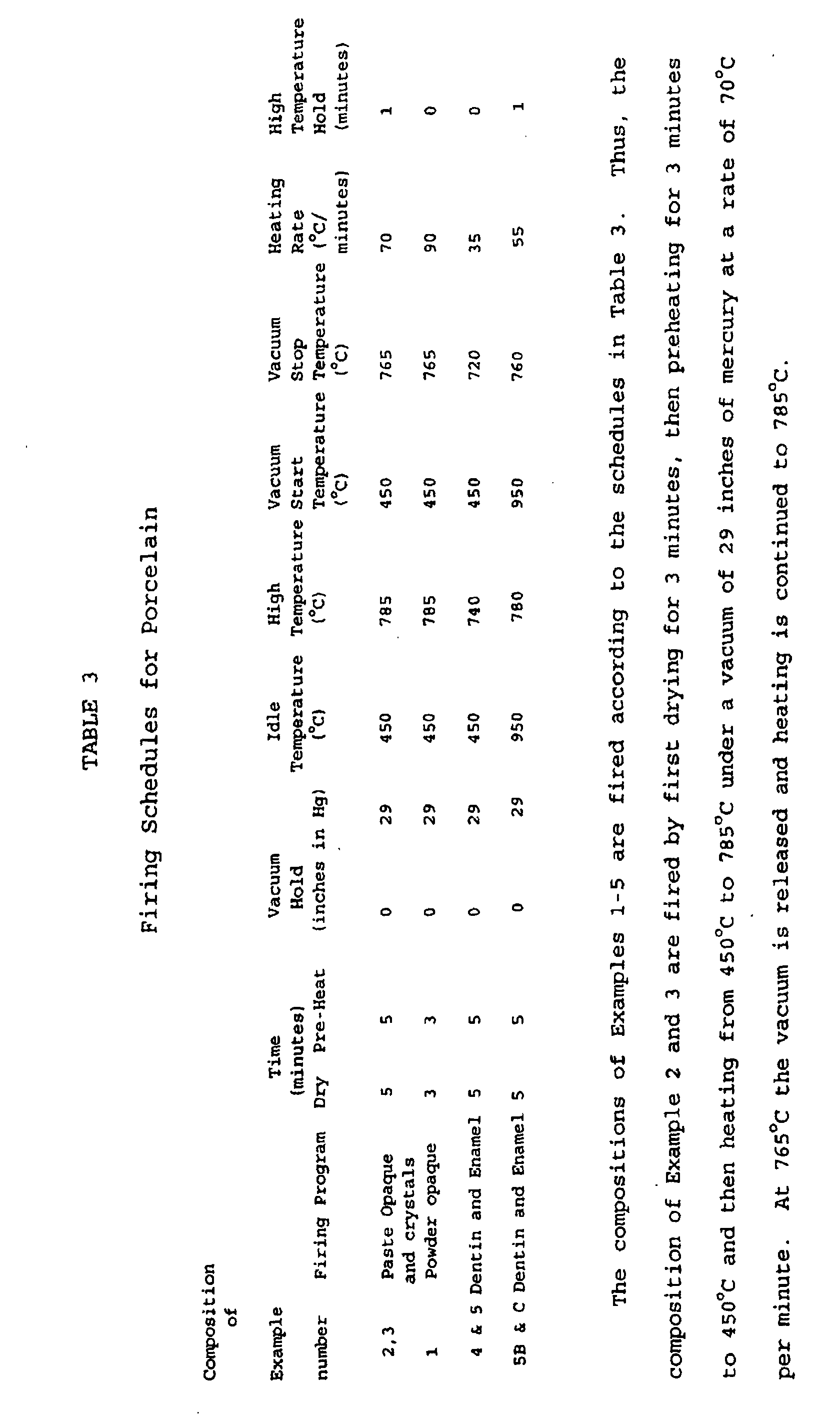

### Example 6

A powder opaque kit container is provided which supports and encloses eighteen bottles each containing 15 g of the product of Example 1 pigmented in sixteen Lumin™ of VITA Zahnfabrik shades, one AO shade and one BO shade; nine bottles of modifier porcelain each containing 15 g of the composition of Example 1A and pigmented White, Gray, Pink, Violet, Ochre, Yellow, Tan, Orange or Sienna; a bottle containing 120 ml of carving Liquid, sold by Ceramco, Inc.; and two Finesse Opaque Shade Fans. Each bottle has a threaded body and a threaded cap. Each shade fan has eighteen porcelain chips. Each chip represents the shade of contents of one of the bottles containing one of the pigmented compositions of Examples 1 and 1A after firing as shown in Table 3.

### Example 7

A paste opaque kit container is provided which supports and encloses eighteen syringes each having 2 ml of paste opaque porcelain, prepared as disclosed in Example 2 and pigmented in sixteen Lumin™ of VITA Zahnfabrik shades, one AO shade and one BO shade; nine bottles of modifier porcelain each having 3 ml of the composition of Example 2A, mixed with gel and pigmented White, Gray, Pink, Violet, Ochre, Yellow, Tan, Orange, or Sienna; a bowl; a bottle containing 10 g of crystals having the composition of Example 3; a brush for application of the pastes; a bottle containing 15 ml of Opaque Modifier Liquid, sold by Ceramco, Inc.; and two Opaque Shade Fans. Each shade fan has porcelain chips. Each chip represents one of the compositions of Example 2 and Example 2A in the eighteen syringes and nine bottles after being dispensed and then fired as shown in Table 3.

### Example 8

A prosthesis is prepared by forming an alloy metal into a dental tooth crown shape and oxidizing or degassing alloy according to the alloy manufacturer's instructions. The powder opaque porcelain composition of Example 1 is then mixed with distilled water to a thin paste like consistency. The opaque paste is painted onto the alloy in a thin even layer, and condensed slightly to form an even coating on the outer surface of the alloy. The coated alloy is then fired according to Table 3. A second coat of powder opaque composition of Example 1 is then painted onto the alloy metal to completely mask it. Opaque modifiers are then applied. After condensing slightly to form an even coating outer surface the coated alloy is fired according to Table 3. The prosthesis product has a slight sheen after firing.

### Example 8A

To 1 part of the product of Example 8 is applied, 2 parts of the product Example 4 having the composition shown in Table 1 and then 1 part of the product of Example 5 from Table 1. This coating is then fired accordingly to Table 3 under Example 4.

### Example 9

A prosthesis is prepared by forming an alloy metal into a dental bridge shape, oxidizing or degassing the alloy according to the alloy manufacturer's instructions. Using a brush, the opaque paste composition of Example 2 is applied to the alloy in a thin even layer. The brush coated alloy is dried at 23°C for 10 minutes and then fired according to Table 3. A second coat of the paste opaque porcelain paste composition of Example 2 is applied to the alloy metal to completely mask it. Opaque modifiers are then applied. Crystals are then sprinkled onto the second layer of the paste opaque and the coated alloy dental bridge restoration is fired according to Table 3. The restoration has a sandpaper or emery board appearance after firing.

### Example 9A

To 1 part of the product of Example 9 is applied, 2 parts of the product Example 4 having the composition shown in Table 1 and then 1 part of the product of Example 5 from Table 1. This coating is then fired accordingly to Table 3 under Example 9A.

## Claims

1. A low-fusing temperature porcelain composition for use in a dental prosthesis, comprising,
40 to 65 percent by weight SiO₂,
6 to 12 percent by weight Al₂ O₃,
6 to 12 percent by weight Na₂O,
5.5 to 10.5 percent by weight K₂O,
1 to 3 percent by weight Li₂O,
0.8 to 2.5 percent by weight CaO,
0.5 to 4 percent by weight B₂O₃,
0.1 to 0.8 percent by weight CeO₂, and further comprising
(a) 0.01 to 3.0 percent by weight P₂O₅ whereby the composition has a coefficient of thermal expansion of from 11.5 to 12.5 ppm °K⁻¹ 30° to 430 °C; or
(b) 0.5 to 2.0 percent by weight Tb₂O₃, and 1 to 3 percent by weight MgO; or
(c) at least 0.4 percent by weight of Y₂ O₃.

2. The porcelain of claim 1, having a fusing temperature of less than 800° C and a coefficient of thermal expansion compatible with a substrate of metal or ceramic suitable for a dental prosthetic.

3. A low-fusing temperature porcelain composition for use in a dental prosthesis, comprising,
60 to 65 percent by weight SiO₂,
8 to 12 percent by weight Al₂O₃,
7 to 12 percent by weight Na₂O,
5.5 to 9.0 percent by weight K₂O,
1.5 to 3 percent by weight Li₂O,
1.2 to 2.5 percent by weight CaO,
0.5 to 4 percent by weight B₂O₃,
0.1 to 0.6 percent by weight CeO₂.

4. The porcelain of claim 3 further comprising
(d) 0.5 to 3 percent by weight of MgO; or
(e) 0.5 to 1. 5 percent by weight of Tb₂O₃; or
(f) 10 to 20 percent by weight ZrO₂; or
(g) 5 to 10 percent by weight Y₂O₃-SiO₂.

5. A low-fusing temperature porcelain composition for use in a dental prosthesis, comprising
40 to 47 percent by weight SiO₂,
5 to 8 percent by weight Al₂O₃,
6 to 12 percent by weight Na₂O,
9.0 to 10.5 percent by weight K₂O,
1 to 3 percent by weight Li₂O,
0.8 to 3.0 percent by weight CaO,
0.8 to 2.0 percent by weight B₂O₃ and
0.3 to 1.0 percent by weight CeO₂.

6. The composition according to claim 5, comprising
6 to 8 percent by weight Al₂O₃,
8 to 12 percent by weight Na₂O,
1 to 2 percent by weight Li₂O,
0.8 to 1.5 percent by weight CaO,
0.8 to 1.5 percent by weight B₂O₃, and
0.3 to 0.8 percent by weight CeO₂.

7. A glass frit composition having a processing temperature of about 705°C and a coefficient of thermal expansion of from about 12 to 13 ppm °K⁻¹ [20 to 500°C] and having a composition as defined in any one of claims 1 to 6.

8. The composition of claim 7, fused to a ceramic substrate.

9. The composition of claim 7, fused to a substrate of metal or ceramic.

10. The composition of claim 7, wherein said composition is as defined in claim 3.

11. The composition of claim 7, wherein said composition wherein said composition is as defined in claim 6.

12. A kit-of-parts comprising at least three containers supported by a container support, wherein each container contains a composition as defined in any one of claims 1 to 6.

13. A method of making a dental prosthesis, comprising:
providing and shaping a low-fusing temperature porcelain composition for use in a dental prosthesis as defined in any one of claims 1 to 7,
and heating said composition to between 700° C and 760° C to make a dental prosthesis.

14. The method according to claim 13, wherein the glass frit is fused to a ceramic substrate to form the dental prosthesis.

15. The method according to claim 13, wherein the glass frit is fused to a ceramic or metal substrate to form the dental prosthesis.

16. The method of claim 13 wherein said composition is as defined in claim 3.

## Patentansprüche

1. Niedrig schmelzende Porzellan-Zusammensetzung zum Einsatz in einer Dentalprothese, umfassend
40 bis 65 Gew.-% SiO₂,
6 bis 12 Gew.-% Aℓ₂O₃,
6 bis 12 Gew.-% Na₂O,
5,5 bis 10,5 Gew.-% K₂O,
1 bis 3 Gew.-% Li₂O,
0,8 bis 2,5 Gew.-% CaO,
0,5 bis 4 Gew.-% B₂O₃,
0,1 bis 0,8 Gew.-% CeO₂ und weiter umfassend
(a) 0,01 bis 3,0 Gew.-% P₂O₅, wobei die Zusammensetzung einen Koeffizienten der Wärmeausdehnung von 11,5 bis 12,5 ppm °K⁻¹ von 30 bis 430°C aufweist, oder
(b) 0,5 bis 2,0 Gew.-% Tb₂O₃ und 1 bis 3 Gew.-% MgO oder
(c) mindestens 0,4 Gew.-% Y₂O₃.

2. Porzellan nach Anspruch 1 mit einer Schmelztemperatur von weniger als 800°C und einem Koeffizienten der Wärmeausdehnung, der mit einem für eine Dentalprothese geeigneten Substrat aus Metall oder Keramik verträglich ist.

3. Niedrig schmelzende Porzellan-Zusammensetzung zum Einsatz in einer Dentalprothese, umfassend
60 bis 65 Gew.-% SiO₂,
8 bis 12 Gew.-% Aℓ₂O₃,
7 bis 12 Gew.-% Na₂O,
5,5 bis 9,0 Gew.-% K₂O,
1,5 bis 3 Gew.-% Li₂O,
1,2 bis 2,5 Gew.-% CaO,
0,5 bis 4 Gew.-% B₂O₃,
0,1 bis 0,6 Gew.-% CeO₂.

4. Porzellan nach Anspruch 3, weiter umfassend
(d) 0,5 bis 3 Gew.-% MgO oder
(e) 0,5 bis 1,5 Gew.-% Tb₂O₃ oder
(f) 10 bis 20 Gew.-% ZrO₂ oder
(g) 5 bis 10 Gew.-% Y₂O₃-SiO₂.

5. Niedrig schmelzende Porzellan-Zusammensetzung zum Einsatz in einer Dentalprothese, umfassend
40 bis 47 Gew.-% SiO₂,
5 bis 8 Gew.-% Aℓ₂O₃,
6 bis 12 Gew.-% Na₂O,
9,0 bis 10,5 Gew.-% K₂O,
1 bis 3 Gew.-% Li₂O,
0,8 bis 3,0 Gew.-% CaO,
0,8 bis 2,0 Gew.-% B₂O₃ und
0,3 bis 1,0 Gew.-% CeO₂.

6. Zusammensetzung nach Anspruch 5, umfassend
6 bis 8 Gew.-% Aℓ₂O₃,
8 bis 12 Gew.-% Na₂O,
1 bis 2 Gew.-% Li₂O,
0,8 bis 1,5 Gew.-% CaO,
0,8 bis 1,5 Gew.-% B₂O₃ und
0,3 bis 0,8 Gew.-% CeO₂.

7. Glasfritten-Zusammensetzung mit einer Verarbeitungs-Temperatur von etwa 705°C und einem Koeffizienten der Wärmeausdehnung von etwa 12 bis 13 ppm °K⁻¹ [20 bis 500°C] und mit einer Zusammensetzung, wie sie in einem der Ansprüche 1 bis 6 definiert ist.

8. Zusammensetzung nach Anspruch 7, geschmolzen an ein Keramiksubstrat.

9. Zusammensetzung nach Anspruch 7, geschmolzen an ein Substrat aus Metall oder Keramik.

10. Zusammensetzung nach Anspruch 7, worin die Zusammensetzung wie in Anspruch 3 definiert ist.

11. Zusammensetzung nach Anspruch 7, worin die Zusammensetzung wie in Anspruch 6 definiert ist.

12. Ausstattung von Teilen, umfassend mindestens drei durch einen Behälterträger getragene Behälter, worin jeder Behälter eine Zusammensetzung enthält, wie sie in einem der Ansprüche 1 bis 6 definiert ist.

13. Verfahren zum Herstellen einer Dentalprothese, umfassend:
Bereitstellen und Formen einer niedrig schmelzenden Porzellan-Zusammensetzung zum Einsatz in einer Dentalprothese, wie sie in irgendeinem der Ansprüche 1 bis 7 definiert ist, und
Erhitzen dieser Zusammensetzung auf zwischen 700°C und 760°C, um eine Dentalprothese herzustellen.

14. Verfahren nach Anspruch 13, worin die Glasfritte zur Bildung der Dentalprothese an ein Keramik-Substrat geschmolzen wird.

15. Verfahren nach Anspruch 13, worin die Glasfritte zur Bildung der Dentalprothese an ein Keramik- oder Metall-Substrat geschmolzen wird.

16. Verfahren nach Anspruch 13, worin die Zusammensetzung wie in Anspruch 3 definiert ist.

## Revendications

1. Composition de porcelaine à basse température de fusion destinée à être utilisée dans une prothèse dentaire, comprenant,
40 à 65 % en poids de SiO₂,
6 à 12 % en poids de Al₂O₃,
6 à 12 % en poids de Na₂O,
5,5 à 10,5 % en poids de K₂O,
1 à 3 % en poids de Li₂O,
0,8 à 2,5 % en poids de CaO,
0,5 à 4 % en poids de B₂O₃,
0,1 à 0,8 % en poids de CeO₂, et comprenant en outre
(a) 0,01 à 3,0 % en poids de P₂O₅, la composition ayant ainsi un coefficient de dilatation thermique compris dans l'intervalle de 11,5 à 12,5 ppm °K⁻¹ [30° à 430°C] ; ou
(b) 0,5 à 2,0 % en poids de Tb₂O₃, et 1 à 3 % en poids de MgO ; ou
(c) au moins 0,4 % en poids de Y₂O₃.

2. Porcelaine suivant la revendication 1, ayant une température de fusion inférieure à 800°C et un coefficient de dilatation thermique compatible avec un substrat constitué d'un métal ou d'une matière céramique convenable pour une prothèse dentaire.

3. Composition de porcelaine à basse température de fusion destinée à être utilisée dans une prothèse dentaire, comprenant
60 à 65 % en poids de SiO₂,
8 à 12 % en poids de Al₂O₃,
7 à 12 % en poids de Na₂O,
5,5 à 9,0 % en poids de K₂O,
1,5 à 3 % en poids de Li₂O,
1,2 à 2,5 % en poids de CaO,
0,5 à 4 % en poids de B₂O₃,
0,1 à 0,6 % en poids de CeO₂.

4. Porcelaine suivant la revendication 3, comprenant en outre
(d) 0,5 à 3 % en poids de MgO ; ou
(e) 0,5 à 1,5 % en poids de Tb₂O₃ ; ou
(f) 10 à 20 % en poids de ZrO₂ ; ou
(g) 5 à 10 % en poids de Y₂O₃-SiO₂.

5. Composition de porcelaine à basse température de fusion destinée à être utilisée dans une prothèse dentaire comprenant,
40 à 47 % en poids de SiO₂,
5 à 8 % en poids de Al₂O₃,
6 à 12 % en poids de Na₂O,
9,0 à 10,5 % en poids de K₂O,
1 à 3 % en poids de Li₂O,
0,8 à 3,0 % en poids de CaO,
0,8 à 2,0 % en poids de B₂O₃, et
0,3 à 1,0 % en poids de CeO₂.

6. Composition suivant la revendication 5, comprenant
6 à 8 % en poids de Al₂O₃,
8 à 12 % en poids de Na₂O,
1 à 2 % en poids de Li₂O,
0,8 à 1,5 % en poids de CaO,
0,8 à 1,5 % en poids de B₂O₃, et
0,3 à 0,8 % en poids de CeO₂.

7. Composition de fritte de verre ayant une température de traitement d'environ 705°C et un coefficient de dilatation thermique d'environ 12 à 13 ppm °K⁻¹ [20 à 500°C] ayant une composition répondant à la définition suivant l'une quelconque des revendications 1 à 6.

8. Composition suivant la revendication 7, fusionnée à un substrat céramique.

9. Composition suivant la revendication 7, fusionnée à un substrat constitué d'un métal ou d'une matière céramique.

10. Composition suivant la revendication 7, ladite composition répondant à la définition suivant la revendication 3.

11. Composition suivant la revendication 7, ladite composition répondant à la définition suivant la revendication 6.

12. Kit de pièces comprenant au moins trois récipients portés par un support de récipients, dans lequel chaque récipient contient une composition répondant à la définition suivant l'une quelconque des revendications 1 à 6.

13. Procédé pour la production d'une prothèse dentaire, comprenant les étapes consistant :
à prendre et façonner une composition de porcelaine à basse température de fusion destinée à être utilisée dans une prothèse dentaire, répondant à la définition suivant l'une quelconque des revendications 1 à 7,
et à chauffer ladite composition à une température comprise dans l'intervalle de 700°C à 760°C pour produire une prothèse dentaire.

14. Procédé suivant la revendication 13, dans lequel la fritte de verre est fusionnée à un substrat céramique pour former la prothèse dentaire.

15. Procédé suivant la revendication 13, dans lequel la fritte de verre est fusionnée à un substrat céramique ou métallique pour former la prothèse dentaire.

16. Procédé suivant la revendication 13, dans lequel ladite composition répond à la définition suivant la revendication 3.
